# EUROPEAN PATENT APPLICATION

(11) **EP 0 779 062 A1**
(43) Date of publication of application: **18.06.1997**
(21) Application number: 96203456.7
(22) Date of filing: 06.12.1996
(51) Int. Cl.: A61F 2/06, A61M 25/10

(54) **Method for forming a stent and a tubular element and catheter to be used in conjuction therewith**

(30) Priority: 12.12.1995 NL 1001878
(71) Applicant: Cordis Corporation, Miami Lakes Florida 33014 (US)
(72) Inventor: Glastra, Hendrik, 7535 PG Enschede (NL); Haan, Marcel, 9301 EA Roden (NL)
(74) Representative: 't Jong, Bastiaan Jacobus

(57) **Abstract**

This invention relates to a method for forming a stent, comprising providing a tubular element with a central-section and two end-sections placed opposite each other and expanding at least one end-section. The invention also relates to a catheter to be used with for instance the method referred to, comprising a tube-like basic body with a proximal and a distal end, an inflatable, preformed balloon member arranged at the distal end with a shape comprising in inflated state a substantially cylindrical central-section and at least one end-section diverging so as to have a larger diameter.

## Description

The invention relates to a method for forming a stent.

It is known to form a stent by expanding a tubular element. This is usually done inside the body of the patient. The tubular element is introduced by means of a catheter in for instance a blood vessel of which the wall requires the support of a stent. As soon as the tubular element has been manoeuvred into the correct position by means of the catheter, the tubular element is expanded, for instance by inflating a balloon inserted into the tubular element.

The object of the invention is to improve such a method.

This is achieved by providing, with the method for forming a stent, a tubular element comprising a central-section and two end-sections placed opposite each other, of which at least one end-section will be expanded. The expanded end-section ensures that the stent will be positioned securely in the target-position. Especially when two end-sections are expanded in relation to the central-section, the stent can be employed in a suitable manner at the site of a constriction in for instance a blood vessel. This constriction may be caused for instance by a tumour in the surrounding tissue. After forming the stent it will remain properly in position, as the expanded end-sections are placed on either side of the constriction.

A suitable embodiment of the invention is characterised in claim 2. The method is based on a substantially cylindrical element of which the ends are expanded more than the central- section. A suitable embodiment is additionally characterised in claim 3. The initial, partial expansion of the end-section or the end-sections can be done in advance, whereby in a subsequent step, during which the tubular element is expanded along its entire length, the ultimate shape of the stent is obtained.

A very suitable embodiment of the method is characterised in claim 4.

When in addition the preferred measure as set out in claim 5 is employed, the tubular element, in substantially cylindrical shape, can be used as basic material, whereas the desired shape with the expanded end-sections can be brought about by inflating the balloon. The at least one end-section of the balloon, diverging so as to have the larger diameter, forms the relatively expanded end-section of the stent.

The invention relates to and also provides a tubular element to be used with a method according to the invention. The tubular element is characterised in claim 7.

Preferably the measure as set out in claim 8 is employed. As the end-section diverges uniformly, good contact between a stent formed of this tubular section and the surrounding structure can be achieved.

The invention relates to and also provides a catheter to be used with a method according to the invention. This catheter comprises a tube-like basic body with a proximal and a distal end, an inflatable, preformed balloon member arranged at the distal end, of which the shape in the inflated state comprises a substantially cylindrical central-section and at least one end-section diverging so as to have a larger diameter.

It should be noted that where the word 'stent' is used in this text, it is used to refer to any element which is formed of a tubular element by expanding it to a desired diameter and which, following this expansion, makes contact with the walls of a channel, such as for instance a blood vessel and wherein a central channel of this element forms part of said channel.

The invention will be explained in greater detail in the following description with reference to the attached drawings.

Figures 1-4 inclusive show four phases of the method according to a first embodiment of the invention.

Figures 5 and 6 show two phases of another embodiment corresponding to the figures 2 and 3 respectively.

Figure 7 illustrates a tubular element to be used with an alternative embodiment of a method according to the invention.

Figure 8 shows the tubular element 7 after it has been expanded to form a stent.

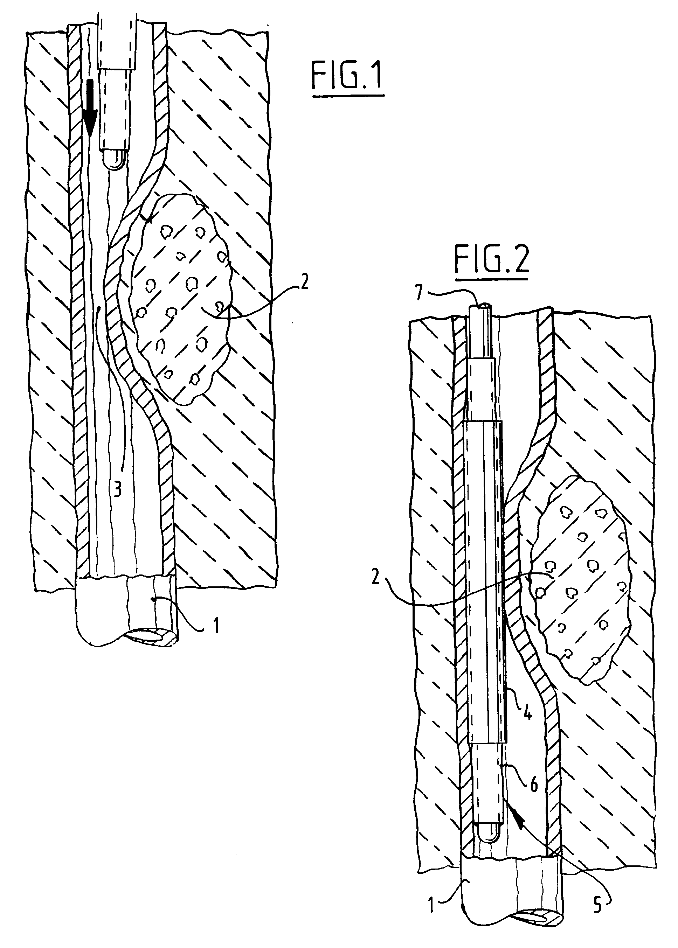

In the figures 1 - 4 inclusive the forming of a stent, with the method according to the invention, is illustrated in an environment in which this stent can function. As can be seen in figure 1, this is a blood vessel 1 which has for instance a constriction 3 due to a tumour 2 in the surrounding tissue.

For the purpose of making the stent, a tubular element 4 is provided. This tubular element 4 has a small diameter and can be manoeuvred by means of a catheter 5, in a manner known as such, to the site of the constriction 3. The catheter 5 has a tube-like basic body 7 at the distal end of which, shown in figure 2, a balloon element 6 has been arranged. The distal end-section of the catheter 5 has been pushed inside the tubular element 4.

After the tubular element 4 has been manoeuvred into the desired position, medium under pressure, for instance a liquid under pressure, is supplied to the internal space 9 of the balloon 6, via a lumen 8 in the tube-like body 7. Via openings 10, this internal space 9 is connected to the lumen 8 of the tube-like basic body 7.

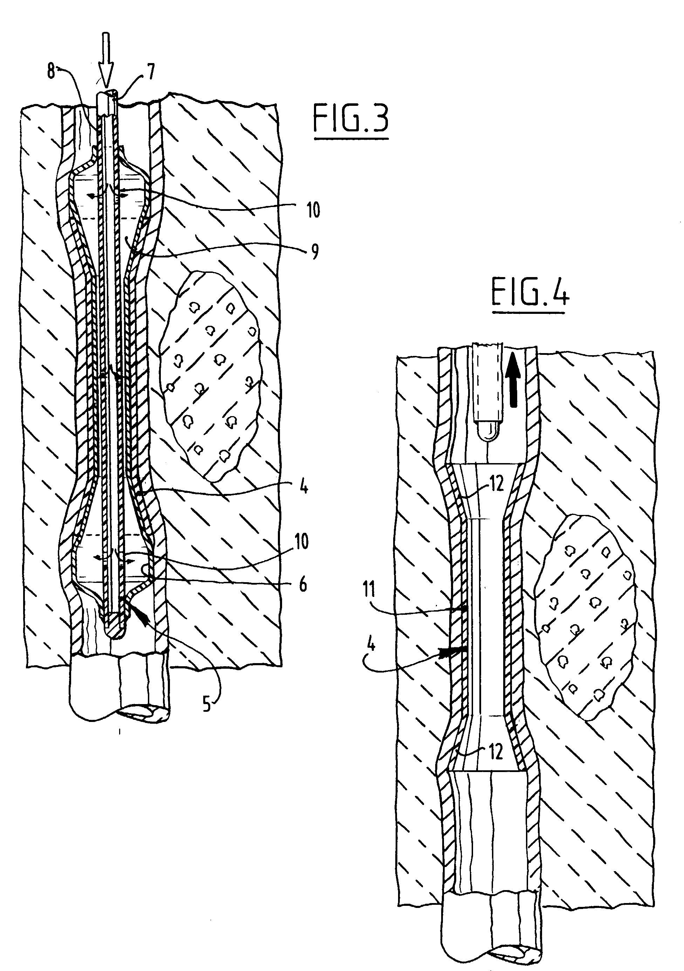

As can be seen in figure 3, the balloon 6 has been preformed and comprises a substantially cylindrical central- section with on either side end-sections diverging so as to have larger diameters. When sufficient pressure has been generated inside the internal space of the balloon, the balloon 6 will inflate to take on the shape illustrated in cross-section in figure 3.

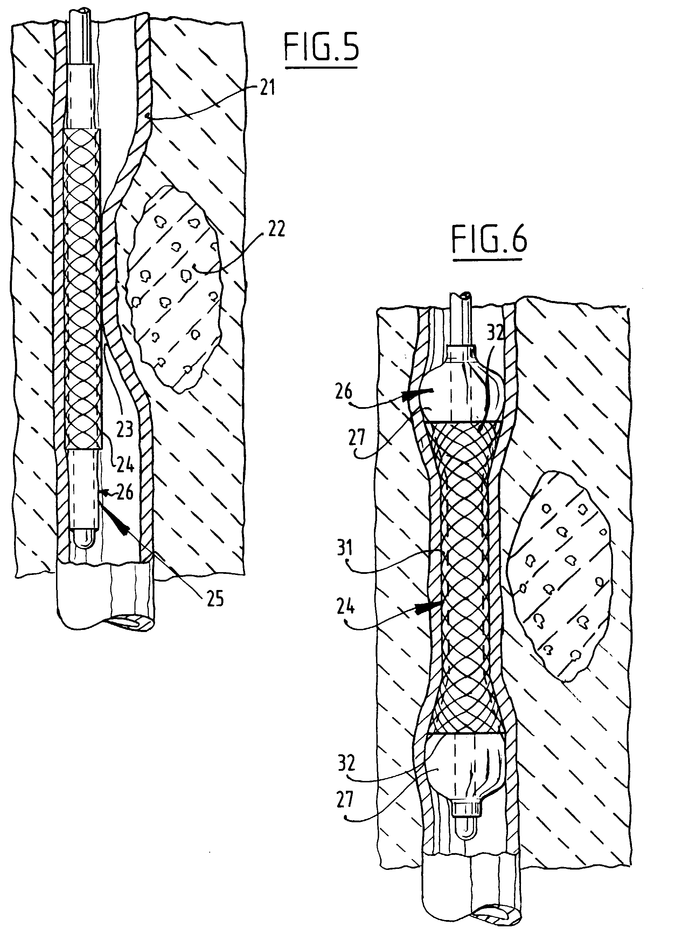

On doing so the tubular element 4 will be expanded to form a stent. Following expansion, the balloon is deflated and the catheter can be removed. Figure 4 illustrates the situation after the stent 4 has been formed and the catheter is being withdrawn.

As can be seen clearly in figure 4, the stent 4 has a substantially cylindrical central-section 11 and two end-sections 12 which have been expanded more. From the central-section 11 the end-sections 12 diverge uniformly so as to have larger diameters.

Due to these funnel-shaped end-sections 12, which are placed on either side of the constriction 3, the entire stent 4 is enclosed inside the constriction 3. In actual practice, the expanded end-sections 12 cannot pass the constriction 3, which ensures locking the stent 4 in position.

With the illustration of the method in the figures 5 and 6 a similar surrounding has been shown as in the figures 1 -4 inclusive. Also here a blood vessel 21 has been shown inside of which a stent has to be formed. This blood vessel 21 has a constriction 23 caused by a tumour 22 in the adjoining tissue.

The tubular element 24 used in accordance with the invention has been made up of metal wire in this case, in particular rustproof steel wire. The use of metal wire in order to form a stent is known as such.

The tubular element 24 is also in this case arranged at the site of the constriction 23 by means of a catheter 25 provided with a balloon 26.

The balloon 26 comprises a cylindrical section and diverging end-sections 27 in a manner corresponding to the one shown in figure 3. By inflating the balloon 26 the tubular element 24 is expanded. The diverging end-sections 27 of the balloon 26 expand the end-sections 32 to a greater degree, as a result of which the stent is formed with a substantially cylindrical central-section 31 and diverging end-sections 32.

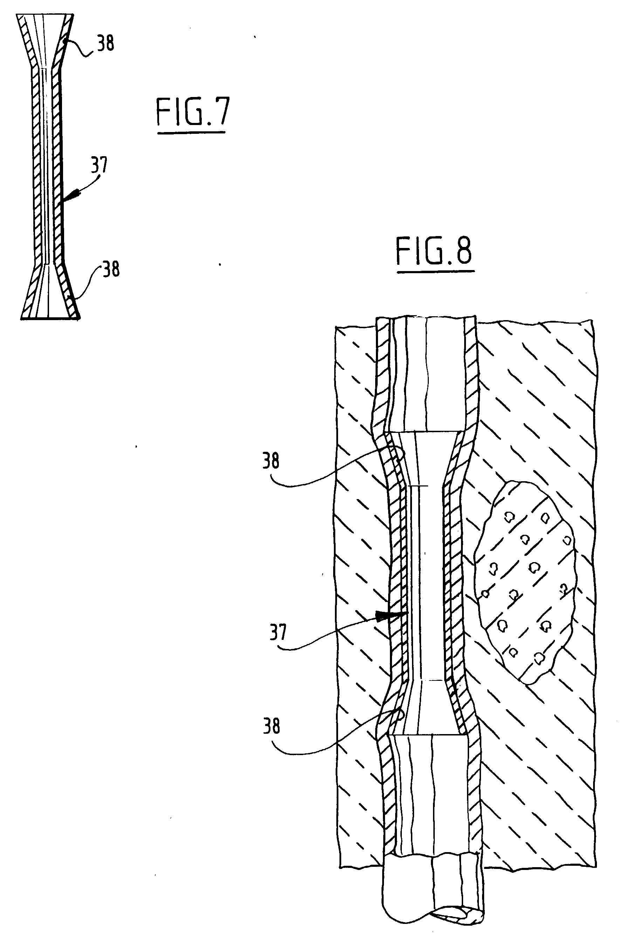

As can be seen in figure 7, the tubular end-section may also have end-sections 38 expanded in advance. The tubular element 37 is arranged in the desired position and can be given the desired shape by expanding it uniformly along its entire length. This form has been illustrated in figure 8. Uniform expansion along the entire length can for instance be effected by means of a balloon catheter which has substantially the same diameter along its entire length. By exerting a uniform pressure along the entire length, a uniform expansion of the tubular element into the stent will be achieved.

Expanding the tubular element into the stent with the desired shape does not necessarily have to be done using a balloon. The tubular element may for instance be a stent compressed along its entire length into a smaller diameter. The tubular element formed in this way can be 'frozen' in the compressed state and be expanded again at the desired location, for instance by supplying warmth or another form of energy. All such variations come within the scope of the attached claims.

## Claims

1. Method for forming a stent comprising the providing of a tubular element with a central-section and two end-sections placed opposite each other and expanding at least one end-section.

2. Method as claimed in claim 1, wherein the tubular element is expanded along its entire length and the end-section is expanded more than the central-section.

3. Method as claimed in claim 2, wherein the end-section is initially expanded partially and the tubular element is expanded more at a later stage along its entire length.

4. Method as claimed in one of the previous claims, furthermore comprising providing a balloon catheter which has been provided at a distal end with an inflatable balloon, arranging a tubular body around the balloon and expanding the tubular element by inflating the balloon.

5. Method as claimed in claim 4, wherein the balloon is provided with a shape comprising a substantially cylindrical central-section and at least one end-section diverging so as to have a larger diameter.

6. Method as claimed in claim 5, wherein the tubular element has been provided with a substantially cylindrical shape.

7. Tubular element to be used with a method as claimed in one of the previous claims, comprising a substantially cylindrical central-section with at least one expanded end-section.

8. Tubular element as claimed in claim 7, wherein the end-section diverges uniformly from the central-section so as to have a larger diameter.

9. Catheter to be used with a method as claimed in one of the claims 5 - 7 inclusive, comprising a tube-like basic body with a proximal and a distal end, an inflatable, preformed balloon member arranged at the distal end with a shape comprising in inflated state a substantially cylindrical central-section and at least one end-section diverging so as to have a larger diameter.
